# EUROPEAN PATENT SPECIFICATION

(11) **EP 0 357 793 B1**
(45) Date of publication and mention of the grant of the patent: **25.05.1994**
(21) Application number: 89903226.2
(22) Date of filing: 09.03.1989
(51) Int. Cl.: A61K 9/16, A61K 9/62, A61K 47/00

(54) **SUSPENDED-RELEASE PREPARATION PREPARED BY USING ALGINATES**
ZUBEREITUNG MIT VERZÖGERTER FREIGABE DURCH VERWENDUNG VON ALGINATEN
PREPARATION A LIBERATION FREINEE PREPAREE GRACE A L'UTILISATION D'ALGINATES

(30) Priority: 09.03.1988 JP 55784/88; 02.09.1988 JP 219747/88
(43) Date of publication of application: 14.03.1990
(73) Proprietor: SNOW BRAND MILK PRODUCTS CO., LTD., Sapporo-shi, Hokkaido 065 (JP)
(72) Inventor: OTAGIRI, Masaki, Kumamoto-shi, Kumamoto 862 (JP); IMAI, Teruko, Kumamoto-shi, Kumamoto 861-21 (JP)
(74) Representative: West, Alan Harry
(86) International application number: PCT/JP89/00255
(87) International publication number: WO 89/08446

(56) References cited:
- EP-A- 0 232 155
- JP-A- 6 144 823
- US-A- 4 401 456
- DERWENT FILE SUPPLIER WPIL, 1985, accession no. 85-247023 [40], Derwent Publications Ltd, London, GB; & JP-A-60 163 811 (WATANABE YAKUHIN KO) 26-08-1985
- CHEMICAL ABSTRACTS, vol. 103, no. 8, 26th August 1985, page 343, abstract no. 59240b, Columbus, Ohio, US; A.A. BADWAN et al.: "A sustained-release drug delivery system using calcium alginate beads", & DRUG DEV. IND. PHARM. 1985, 11(2-3),239-56

## Description

This invention relates to a controlled-release pharmaceutical composition in which a basic medicament is contained in alginate gel beads and to a method for the preparation thereof.

The use of controlled-release pharmaceutical compositions has improved the effectiveness and safety of therapeutic treatments by reducing the number of doses required, sustaining therapeutic concentrations of medicament and reducing systemic and toxic effects as compared with typical rapid-release pharmaceuticals compositions.

In order to control the rate of release of medicaments in vivo, various formulations of pharmaceutical compositions have been employed, for example, microcapsules, nano-capsules and matrices using various natural or synthetic polymers and elastomers. Shigeru Goto and Masakazu Kawada ("New Pharmaceutical Development System General Technology Design" R&D Planning Company, p.140, 1986) describe the preparation of microcapsules or nano-capsules. Furthermore, matrix preparations have been described by M. Bamba et al. (Int. J. Pharmaceut., 2307, 1979) and F. A. Kincl et al. (Archiv. Pharm., 317, 1984) and R. V. Sparer et al. (J. Contr. Release, 1, 23, 1984).

However, these preparations are both complex to prepare and in the choice of the appropriate medicament/polymer combination.

The present invention provides a pharmaceutical composition which is relatively easy to prepare and which exhibits superior release properties. The pharmaceutical of the present invention uses salts of alginic acid (a natural polymer and a constituent of the cell membrane of brown algae) to prepare alginate gel beads.

Alginic acid is commercially available as a sodium salt having various molecular weights. Aqueous solutions of alginic acid are highly viscous, and therefore have been used as a stabilizer or viscous agent in food products such as ice cream, cheese, sherbet and syrup and also used in manufacturing films and fabrics. Alginic acid has to date also been used in the medical and agrochemical fields.

Chemical Abstracts (No. 59240b), Vol. 103, No. 8 [1985] page 343 describes a sustained release drug delivery system using calcium alginate beads.

US 4,401,456 also describes the use of alginate gel beads to provide controlled release of bioactive materials and a process for the preparation thereof.

Accordingly, the present invention provides a controlled-release pharmaceutical composition comprising a basic medicament contained in alginate gel beads, the beads being formed from a sodium salt of alginic acid having a molecular weight of 10,000 to 100,000, a ratio of the number of blocks of mannuronic acid (MM) to the number of blocks of guluronic acid (GG) from 0.13 to 1.77 and a viscosity of a 1% w/v aqueous solution of the sodium salt of less than 100 10⁻³Nm⁻²s (100 cps).

Such a pharmaceutical composition can be prepared by suspending a basic medicament in a sodium salt solution of alginic acid; adding dropwise the resultant suspension to an aqueous solution of calcium chloride; allowing the resultant mixture to stand; and drying the alginate gel beads.

The terms pharmaceutical composition and medicament used hereinafter refers to any composition which comprises a compound suitable for use in the agriculture and agrochemical field, the food industry as well as the medical field for example.

The present invention will now be described with reference to the following examples and accompanying drawings in which:
Fig. 1 shows the release of pindolol from the alginate gel beads of Example 1;
Fig. 2 shows the change in the serum pindolol concentration after oral administration of the alginate gel beads to rabbits;
Fig. 3 shows the change in the serum pindolol concentration after oral administration of the alginate gel beads to beagles;
Fig. 4 shows the absorption of pindolol from alginate gel beads in beagles;
Fig. 5 shows release of nifedipine from the alginate gel beads of Example 2;
Fig. 6 shows the absorption of nifedipine from alginate gel beads;
Fig. 7 shows the effect of the M/G ratio of the alginic acid on the rate of release of drug; and
Fig. 8 shows the change of the serum pindolol concentration in beagles following oral administration of pindolol powder alone and pindolol contained in alginate gel beads having M/G ratio of 1:3.

Examples of the basic medicament which can be used in the present invention include beta-blockers such as pindolol, procaterol, propranolol, pyltelol, and befunolol; calcium antagonists such as nifedipine, verapamil, diltiazem, and nicardipine; antihistamines such as difenhydramine, diphenylpyraline and chlorophenylamine; diuretics such as triamterene and penflutizide, vasodilative agents such as cinnarizine, ifenprodil, and pentoxifylline; and antitusives such as eprazinone, chloroprenaline, chloperastine, trimethoquinol, bromhexine, methoxyphenamine and sulbutanol.

Alginate gel beads containing the above basic medicaments can be prepared using commercially available sodium alginates having a molecular weight of 10,000 - 100,000 and a viscosity of a 1% w/v acqueous solution of the sodium alginate of less than 100 10⁻³Nm⁻²s (100 cps).

The alginate of choice will depend upon the type of medicament used and the rate of release of medicament that is required.

Preferred alginates are as follows:

| Sodium alginate | MM/GG | Viscosity (10⁻³Nm⁻²s) |
|---|---|---|
| No. 1 | 0.13 | 13.3 (13.3cp) |
| No. 2 | 0.66 | 13.7 (13.7cp) |
| No. 3 | 1.77 | 12.1 (12.1cp) |

Release of medicament from the alginate gel beads is affected by the ratio of the number of mannuronic acid residues to the number of guluronic acid residues in the alginate polymer (M/G ratio); by the ratio of the number of blocks of mannuronic acid to the number of blocks of guluronic acid in the alginate polymer (MM/GG ratio); and by the viscosity of the alginate. The term 'block' herinafter denotes a sequence of identical uronic acid residues (M or G).

An increase in the guluronic acid content of the alginate causes the matrix to become so dense that release of the medicament is very slow. Furthermore, when the MM/GG ratio is small, for example 0.13, the release of medicament is slow also.

A basic medicament is suspended in a 4% sodium alginate solution and added dropwise through a nozzle to a 0.1 M CaCl₂ solution. The solution is allowed to stand for 72 hours and the alginate gel beads containing the basic drug are collected by filtration. The alginate gel beads are dried in air for 24 hours and then in vacuo at room temperature for 24 hours.

The molecular weight of the sodium alginate greatly affects both the yield of alginate gel beads formed and the amount of the medicament contained therein. With a view to controlling the release of medicament, alginic acids having a low viscosity ie a low molecular weight are used.

The concentration of the basic drug is determined by considering the amount necessary to achieve the desired release.

According to the present invention, the alginate gel beads containing a basic medicament, can be formulated as necessary, for example, into enteric coated tablets.

Further, a pharmaceutical according to the present invention can be used to control the absorption of sodium in the body, since alginic acid forms gel structures by binding to calcium and thus calcium can be replaced by sodium when the alginic acid is diffused to release the medicament therefrom in the intestine.

A process for the preparation of the controlled-release pharmaceutical composition of the present invention and their releasing effects are described more in detail by the following Examples.

### Example 1

### Preparation of alginate gel beads:

Pindolol was suspended in a 4% sodium alginate solution ( M/G ratio of alginic acid = 0.6) to a concentration of 4% and the resultant suspension added dropwise using a nozzle into a 0.1 M CaCl₂ solution. The resultant mixture was allowed to stand for 72 hours to permit alginate gel beads containing pindolol therein to form.

The gel beads thus obtained were collected by filtration, dried in air for 24 hours and then dried in vacuo for 24 hours at room temeprature. The resultant pharmaceutical composition was subjected to a medicament releasing test, oral administration tests in rabbits or beagles and an absorption test in humans.

The sodium alginates used were as follows:
(1) A product of Kimizu Chemicals, sodium alginate with low viscosity (IL₂), the viscosity (1% solution): 20-50 10⁻³Nm⁻²s (20-50 cps);
(2) A product of Kimizu Chemicals, sodium alginate of ultra low viscosity (ULA), the viscosity (10% solution): 500 10⁻³Nm⁻²s (500 cps);
(3) A product of Wako Pure Chemicals, reagent grade sodium alginate (NA), the viscosity (1%):20 10⁻³Nm⁻²s (20 cps); and
(4) A product of Kibun Food Chemipha, Dack alginic acid (DA-20), the viscosity (1%): 20 10⁻³Nm⁻²s (20 cps).

Further, the pindolol content and yield in alginate gel beads are shown in Table 1.

**Table 1**

| | Pindolol (%) | Recovery (%) |
|---|---|---|
| NA | 47.2 | 78.9 |
| IL₂ | 44.9 | 75.8 |
| ULA | 14.5 | 21.5 |
| DA-20 | 60.2 | 77.5 |

### Medicament releasing test:

Alginate gel beads containing 10 mg of pindolol were suspended in 150 ml of water at 37°C. The suspension was stirred at 150 rpm and the medicament released was measured at given intervals.

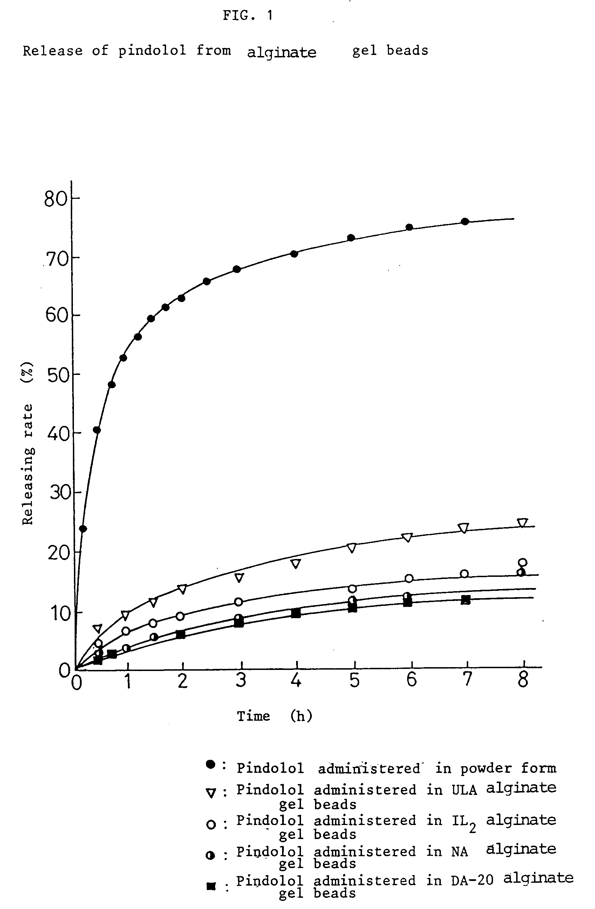

As shown in Fig. 1, the rate of release of pindolol was much slower from the alginate gel beads than from pindolol by itself. Furthermore, an improvement in the release of pindolol was obtained with pharmaceutical compositions prepared from sodium alginates having a low viscosity.

### Oral administration test in rabbits:

Male Japanese white rabbits (2.0 - 2.5 kg) were starved for 24 hours before the administration of medicaments.

Alginate gel beads (30 mg/kg as pindolol) were administered with 100 ml of the alginate gel beads to the test animals and 3 ml of blood taken from the auricular veins of each animals at given intervals. The samples were centrifuged and 1 ml serum was obtained. Pindolol was extracted from the serum and was quantatively measured by high performance liquid chromatography (HPLC).

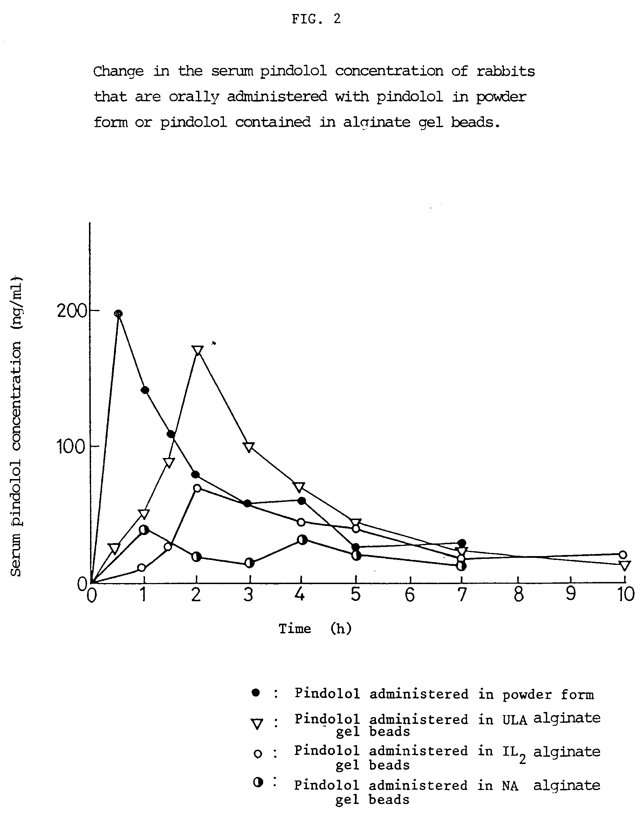

As shown in Fig. 2, the serum pindolol concentration following oral administration was lower in the rabbits administered with pindolol contained in alginate gel beads than in rabbits given pindolol alone. As evident from the results, pindolol contained in the alginate gel beads was absorbed slowly. Moreover, it can be seen that the higher the viscosity of alginic acid, the slower the rate of absorption of pindolol. Accordingly, the pindolol concentration in the serum can be controlled by the choice of alginic acid.

### Oral administration test using beagles:

Male beagles (10 kg) were starved for 24 hours before the adminsitration of a test medicament. Water was given ad libitum.

Pindolol alone (total amount 5 mg/kg) was administered orally as two successive doses of i.e. 2.5 mg/kg with 20 ml of water with a 6 hour interval therebetween. Pindolol contained in alginate gel beads was given orally at 5 mg/kg with 20 ml of water. Water was given ad libitum during the test period. At given intervals, 5 ml of blood was taken from the forefoot vein and centrifuged to provide 2 ml of serum. Pindolol was extracted from the serum and quantitated by high performance liquid chromatography (HPLC).

In this test, alginate gel beads prepared using a low viscosity alginate (IL₂, Kimizu Chemicals) were used.

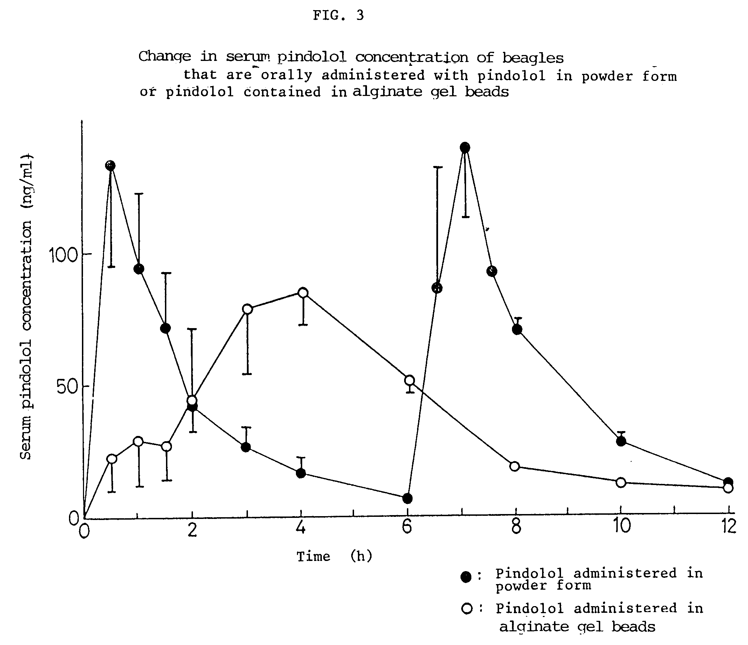

Results are shown in Fig. 3 and Table 2

**Table 2**

| Form of preparation and time elaspsed | AUC (ngh/ml) | MRT (h) | VRT (h²) |
|---|---|---|---|
| Pindolol powder | | | |
| 0 → 6h | 238.0 | 1.74 | 1.74 |
| 0 → 12h | 311.4 | 1.98 | 1.83 |

| Alginate gel beads | | | |
|---|---|---|---|
| 0 → 12h | 445.7 | 4.62 | 5.93 |
| AUC: Concentration in the serum - Area under the time curve MRT: Mean retention time (average retention time of drug in the body the faster the absorption, the smaller the value of MRT.) VRT: Variance of retention time (the longer the retention, the larger the value of VRT). | | | |

The results reveal that pindolol contained in the alginate gel beads was absorbed more slowly and retained activity for a longer period of time than pindolol alone. The controlled-release of pindolol from the alginate gel beads was thus observed.

### Absorption test in humans:

The alginate gel beads (alginic acid having MM/GG = 0.66) which were found to provide the best results in the oral administration test mentioned above, were administered orally to four healthy human adults and compared with a commercially available pindolol slow-releasing pharmaceutical composition, Calvisken (registered trademark, Sankyo Co., Ltd.). Calvisken is a release-retaining nucleated double-layered tablet in which 10 mg of pindolol is contained in the core tablet and the outer layer forms an enteric coat.

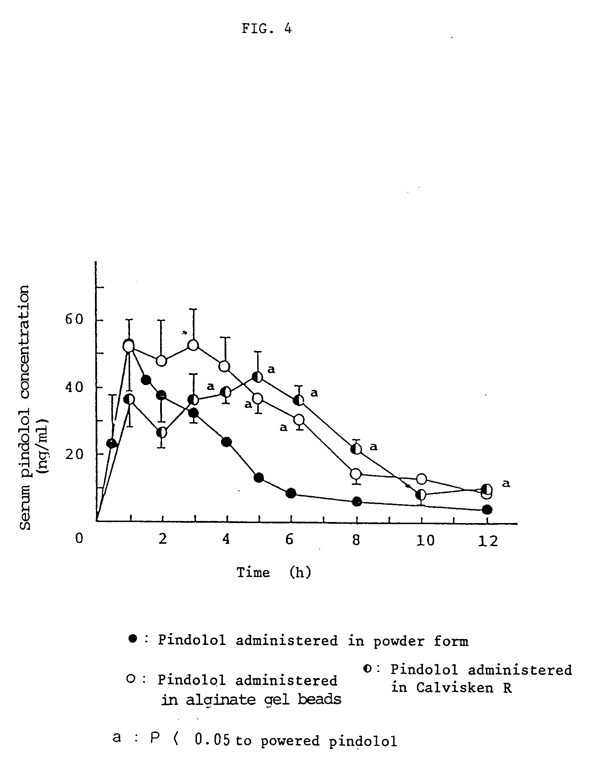

Fig. 4 shows the change in serum pindolol concentration (figures are the average ± standard deviation for the 4 adults). Following oral administration of pindolol powder in an amount equal to half (10 mg) the slow-releasing pharmaceutical composition, Tₘₐₓ ie the time taken to reach the maximum serum concentration, was about 1 hour and thereafter the pindolol concentration gradually decreased. Both controlled-release pharmaceutical compositions maintained a serum pindolol concentration comprable to Cₘₐₓ (maximum serum concentration) for 1 to 6 hours. thus confirming that the alginate gel beads were equally as effective as calvisken R. Since The effective serum concentration pindolol is 10 - 50 ng/ml and that concentration was maintained for 10 hours following the administration of pindolol contained in alginate gel beads. Table 3 shows the changes in serum pindolol concentration.

**Table 3**

| Form of preparation | AUC (ng h/ml) | MRT (h) | VRT (h²) |
|---|---|---|---|
| Pindolol powder | 377.5±48.5* | 3.82±0.32 | 7.44±0.54 |
| Alginate gel beads | 336.3±43.3** | 4.66±0.42^{a)} | 8.16±0.47 |
| Calvisken R | 305.7±25.7** | 5.07±0.30^{a)} | 7.49±0.37 |

| | | | |
|---|---|---|---|
| Note: * AUC₀₋₆ x 2 | | | |
| ** AUC₀₋₁₂ | | | |
| a) p < 0.05 to the value for pindolol powder (Significantly different from that for pindolol powder at the significance level of 5%) | | | |

As shown in Table 3, the AUCs after the administration of the controlled-release pharmaceutical compositions were slightly smaller than 2-fold AUC pindolol alone; however, the difference was not significant. Furthermore, the MRT for the controlled-release pharmaceutical compositions were longer than that for pindolol alone thus, demonstrating their controlled-release properties.

Consequently, it is clear that the alginate gel beads prepared in accordance with example 1 are as effective as the commercially available slow-releasing tablet, Calvisken R.

### Example 2

### Preparation of alginate gel beads:

Nifedipine was suspended in a 4% low viscosity sodium alginate solution (IL₂, Kimizu Chemicals) to a concentration of 4% and the suspension added dropwise using a nozzle into a 0.1 M CaCl₂ solution. The resultant mixture was allowed to stand for 72 hours to permit the alginate gel beads containing nifedipine to form.

The content of nifedipine in the gel beads thus obtained was 45%.

The gel beads were collected by filtration, dried in air for 24 hours and then dried in vacuo for 24 hours at room temperature. The resultant preparation was subjected to the medicament releasing test.

### Medicament releasing test:

The alginate gel beads corresponding to 10 mg as nifedipine were suspended in 150 ml of water at 37°C. The suspension was stirred at 150 rpm and the medicament released measured at given intervals.

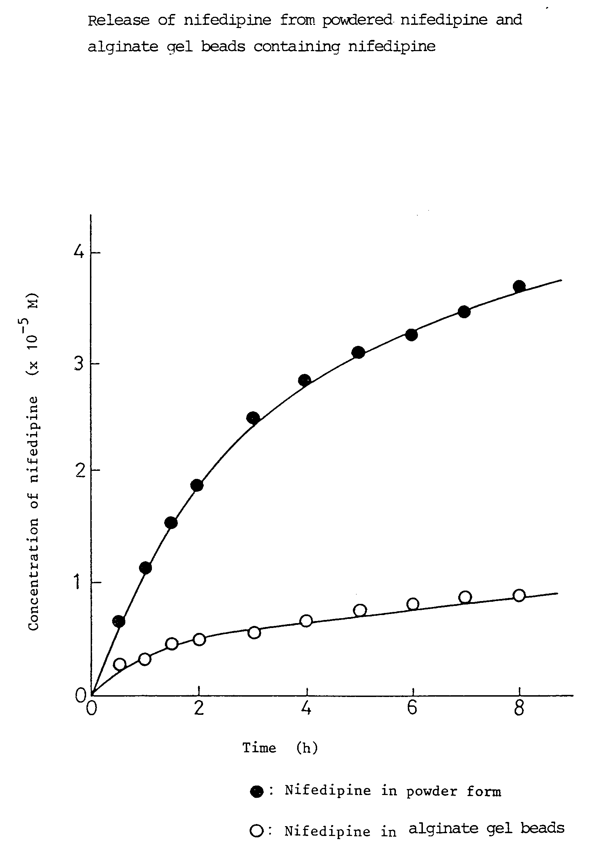

As shown in Fig. 5, the rate of the release of nifedipine was much slower from the alginate gel beads.

### Absorption test in beagles:

Oral administration of nifedipine to beagles was carried out in the same manner as described in Example 1. The nifedipine concentration in the serum was determined in the same manner as described in Example 1.

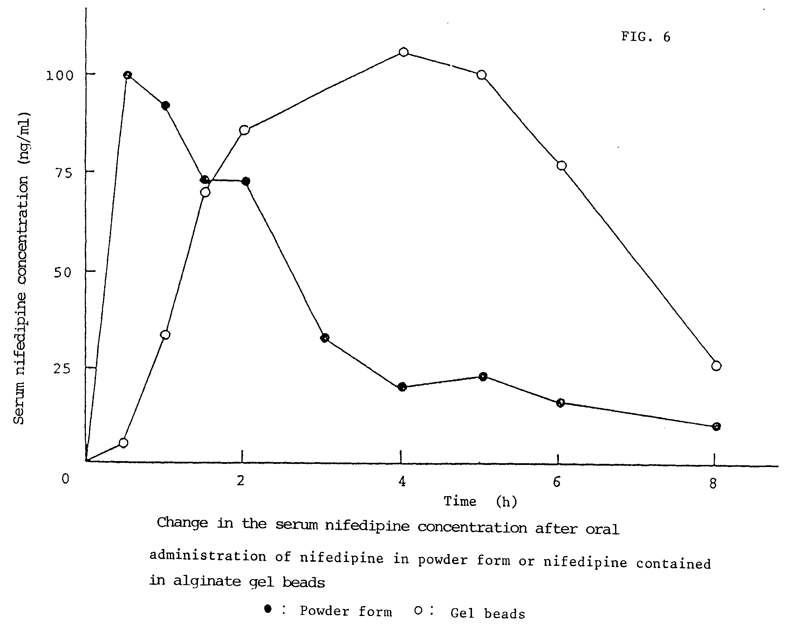

As shown in Fig. 6, nifedipine contained in the alginate gel beads was absorbed more slowly and more extensively than nifedipine in powder form.

### Example 3

This example demonstrates the effect of the ratio of mannuronic and guluronic acid ( M/G ratio) of the alginic acid on the rate of release of medicament from the alginate gel beads.

### Preparation of alginate gel beads:

Sodium alginates having the M/G ratios of 0.5, 1.3 and 2.4 were used. Alginate gel beads were prepared as described in Example 1.

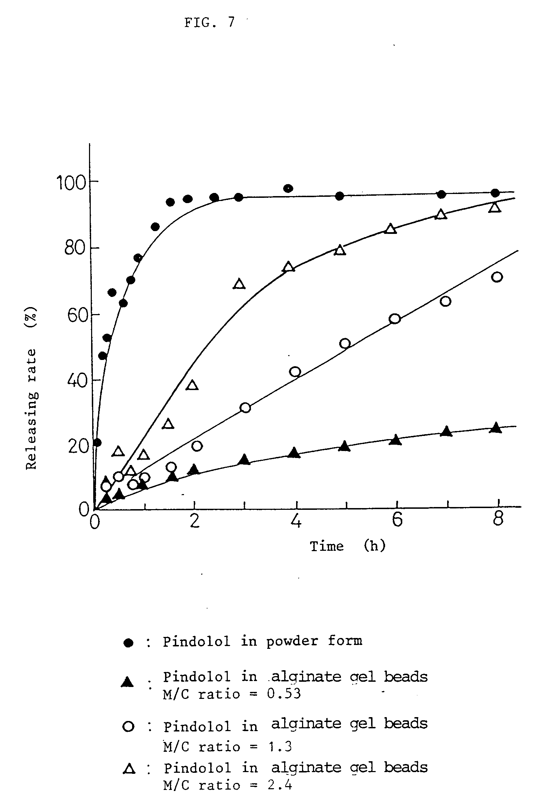

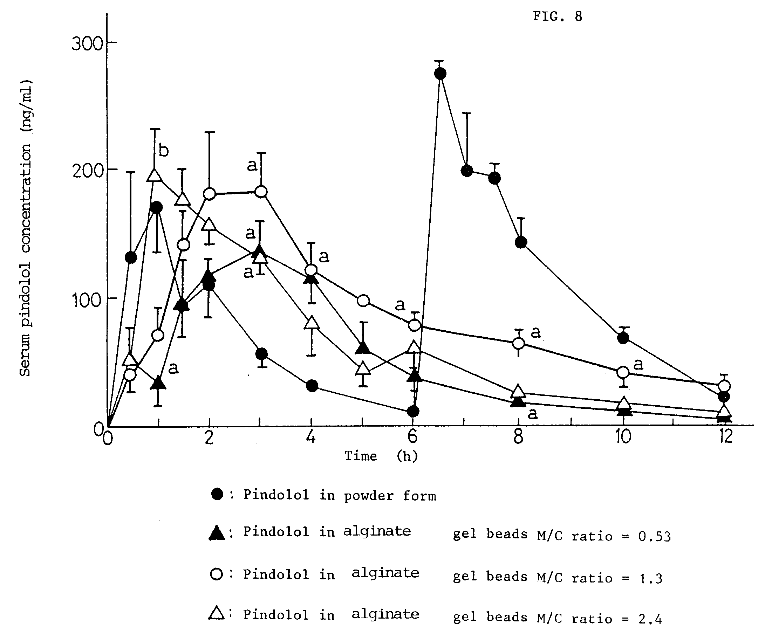

The alginate gel beads thus obtained were subjected to the medicament releasing test (Fig. 7) and the oral administration test (Fig. 8) according to the procedures described in Example 1. As shown in both Fig. 7 and Fig.8 the M/G ratio = 1.3, gave particularly satisfactory results.

A controlled-release pharmaceutical composition of the present invention can be prepared containing a basic medicament in alginate gel beads, in which the M/G ratio, MM/GG ratio and the molecular weight of the alginic acid are selected to give the desired releasing rates. Alginate gel beads having the appropriate matrix structure are easily prepared using a metal ion such as Ca for gel formation.

Furthermore, the pharmaceutical composition of the present invention is particularly useful in the field of medicine. More particularly, it is extremely useful when retention of efficacy, reduction in the number of doses and reduction in systemic and toxic effects are desired.

Furthermore, the controlled-release pharmaceutical composition of the present invention also has utility in various kinds of chemical reactions either as a catalyst or additive. Furthermore, the alginate gel beads may be used in connection with fertilziers, agricultural chemicals, soil chemicals or food additives because the gel beads can be easily formulated into various forms.

## Claims

1. A controlled-release pharmaceutical composition comprising a basic medicament contained in alginate gel beads, the beads being formed from a sodium salt of alginic acid having a molecular weight of 10,000 to 100,000, a ratio of the number of blocks of mannuronic acid (MM) to the number of blocks of guluronic acid (GG) from 0.13 to 1.77 and a viscosity of a 1% w/v aqueous solution of the sodium salt of less than 100 10⁻³Nm⁻²s (100 cps).

2. A controlled-release pharmaceutical composition according to claim 1, wherein the alginate is bound to calcium.

3. A controlled-release pharmaceutical composition according to claim 1 or claim 2, wherein the basic medicament is a beta-antagonist.

4. A controlled-release pharmaceutical composition according to claim 3, wherein the beta-antagonist is pindolol.

5. A controlled-release pharmaceutical composition according to claim 1, wherein the basic medicament is a calcium antagonist.

6. A controlled-release pharmaceutical composition according to claim 5, wherein the calcium antagonist is nifedipine.

7. A method for the preparation of a controlled-release pharmaceutical composition according to claim 1, the method comprising
(a) suspending a basic medicament in a sodium salt solution of alginic acid;
(b) adding dropwise the resultant suspension to an aqueous solution of calcium chloride;
(c) allowing the resultant mixture to stand; and
(d) drying the alginate gel beads.

8. Method according to claim 7, wherein the concentration of basic medicament is 4% w/v of CaCl₂ solution.

9. Method according to claim 7 or claim 8, wherein the mixture is allowed to stand for about 72 hours.

## Patentansprüche

1. Eine pharmazeutische Zubereitung mit gesteuerter Freigabe, die ein Basismedikament umfaßt, das in Alginatgelkügelchen enthalten ist, wobei die Kügelchen aus einem Natriumsalz von Alginsäure mit einem Molekulargewicht von 10.000 bis 100.000, einem Verhältnis der Anzahl von Blöcken von Mannuronsäure (MM) zu der Anzahl von Blöcken von Guluronsäure (GG) von 0,13 bis 1,77 und einer Viskosität einer 1%-igen Gewicht/Volumen wässrigen Lösung des Natriumsalzes von weniger als 100·10⁻³Nm⁻²s (100 Centipoise)gebildet sind.

2. Eine pharmazeutische Zubereitung mit gesteuerter Freigabe nach Anspruch 1, bei der das Alginat an Calcium gebunden ist.

3. Eine pharmazeutische Zubereitung mit gesteuerter Freigabe nach Anspruch 1 oder Anspruch 2, bei der das Basismedikament ein Beta-Antagonist ist.

4. Eine pharmazeutische Zubereitung mit gesteuerter Freigabe nach Anspruch 3, bei der der Beta-Antagonist Pindolol ist.

5. Eine pharmazeutische Zubereitung mit gesteuerter Freigabe nach Anspruch 1, bei der das Basismedikament ein Calcium-Antagonist ist.

6. Eine pharmazeutische Zubereitung mit gesteuerter Freigabe nach Anspruch 5, bei der der Calcium-Antagonist Nifedipin ist.

7. Ein Verfahren für die Herstellung einer pharzeutischen Zubereitung mit gesteuerter Freigabe nach Anspruch 1, wobei das Verfahren umfaßt
(a) daß ein Basismedikament in einer Natriumsalzlösung von Alginsäure suspendiert wird;
(b) tropfenweise die entstandene Suspension zu einer wässrigen Lösung von Calciumchlorid hinzugegeben wird;
(c) die entstehende Mischung stehen gelassen wird und
(d) die Alginatgelkügelchen getrocknet werden.

8. Verfahren nach Anspruch 7, bei dem die Konzentration des Basismedikaments 4 % Gewicht/Volumen CaCl₂-Lösung ist.

9. Verfahren nach Anspruch 7 oder Anspruch 8, bei dem die Mischung etwa 72 Stunden stehen gelassen wird.

## Revendications

1. Une composition pharmaceutique à libération contrôlée comprenant un médicament de base contenu dans des perles de gel d'alginate, les perles étant formées à partir d'un sel de sodium d'acide alginique ayant un poids moléculaire de 10 000 à 100 000, un rapport du nombre des séquences d'acide mannuronique (MM) au nombre des séquences d'acide guluronique (GG) de 0,13 à 1,77 et une viscosité d'une solution aqueuse du sel de sodium à 1 % p/v inférieure à 100 × 10⁻³ Nm⁻²s (100 cP).

2. Une composition pharmaceutique à libération contrôlée selon la revendication 1, dans laquelle l'alginate est lié au calcium.

3. Une composition pharmaceutique à libération contrôlée selon la revendication 1 ou la revendication 2, dans laquelle le médicament de base est un β-antagoniste.

4. Une composition pharmaceutique à libération contrôlée selon la revendication 3, dans laquelle le β-antagoniste est le pindolol.

5. Une composition pharmaceutique à libération contrôlée selon la revendication 1, dans laquelle le médicament de base est un antagoniste du calcium.

6. Une composition pharmaceutique à libération contrôlée selon la revendication 5, dans laquelle l'antagoniste du calcium est la nifédipine.

7. Un procédé pour la préparation d'une composition pharmaceutique à libération contrôlée selon la revendication 1, lequel procédé consiste à
(a) mettre en suspension un médicament de base dans une solution de sel de sodium d'acide alginique ;
(b) ajouter goutte à goutte la suspension obtenue à une solution aqueuse de chlorure de calcium ;
(c) laisser reposer le mélange obtenu ; et
(d) sécher les perles de gel d'alginate.

8. Procédé selon la revendication 7, dans lequel la concentration du médicament de base est de 4 % p/v de la solution de CaCl₂.

9. Procédé selon la revendication 7 ou la revendication 8, dans lequel on laisse le mélange reposer pendant environ 72 heures.
